# EUROPEAN PATENT SPECIFICATION

(11) **EP 4 225 149 B1**
(45) Date of publication and mention of the grant of the patent: **12.08.2026**
(21) Application number: 21807240.3
(22) Date of filing: 05.10.2021
(51) Int. Cl.: A61B 8/08, A61B 8/00

(54) **ULTRASOUND IMAGE PROCESSING SYSTEM AND METHOD**
ULTRASCHALLBILDVERARBEITUNGSSYSTEM UND -VERFAHREN
SYSTÈME ET PROCÉDÉ DE TRAITEMENT D'IMAGE ULTRASONORE

(30) Priority: 05.10.2020 IT 202000023353
(43) Date of publication of application: 16.08.2023
(73) Proprietor: Costanzia S.r.l., 20900 Monza (IT)
(72) Inventor: ROTILIO, Alessandro Carmelo, 20132 Milano (IT); SOLBIATI, Marco, 20154 Milano (IT)
(74) Representative: Penza, Giancarlo
(86) International application number: PCT/IB2021/059109
(87) International publication number: WO 2022/074550

(56) References cited:
- EP-A1- 3 530 221
- US-A1- 2016 354 152
- Y SATO ET AL: "Image guidance of breast cancer surgery using 3-D ultrasound images and augmented reality visualization", IEEE TRANSACTIONS ON MEDICAL IMAGING, 2 January 1998 (1998-01-02), UNITED STATES, pages 681 - 693, XP055468407, Retrieved from the Internet <URL:https://ieeexplore.ieee.org/ielx5/42/15866/00736019.pdf?tp=&arnumber=736019&isnumber=15866> [retrieved on 20210610], DOI: 10.1109/42.736019
- MARCO SOLBIATI ET AL: "Augmented reality for interventional oncology: proof-of-concept study of a novel high-end guidance system platform", EUROPEAN RADIOLOGY EXPERIMENTAL, vol. 2, no. 1, 31 July 2018 (2018-07-31), XP055769915, DOI: 10.1186/s41747-018-0054-5

## Description

### FIELD OF APPLICATION

The present invention relates to a system for processing ultrasound images.

More in particular, the present invention relates to a system for processing ultrasound images in interaction with a medical instrument for treating and diagnosing pathologies.

### PRIOR ART

Standard ultrasound examinations are known which have been developed to enable a medical operator to intervene with medical instruments in order to treat and diagnose pathologies, such as, for example, a needle for amniocentesis/biopsy, simultaneously with the ultrasound examination.

An operator performing an ultrasound examination positions an ultrasound probe on a patient's body in the position closest to the area in which he or she will have to intervene with the instrument, so that the ultrasound image produced on the ultrasound beam plane is displayed on a monitor, and performs the function of guiding the medical operator during the intervention.

The operator who has to intervene on the patient inserts an instrument into the body following a path deduced from the ultrasound image produced and visible on the monitor; the operator inserts the instrument into the body along the plane of the ultrasound beam, according to practice, in order to clearly visualize the needle and be able to act in an optimal manner.

A serious drawback of this approach is that when the instrument is not perfectly aligned with the ultrasound plane, the instrument is only partially visible on the monitor, and the visibility is often reduced to a single point of intersection with the ultrasound plane, making its use complex and dangerous.

The incomplete visibility of the instrument could cause an interference of the instrument itself with organs and tissues along the path of access to the organ and with the tissue that is the target for performing the diagnostic or therapeutic function for which the instrument is intended.

There is a strong need to improve the interaction between an instrument and the practice of ultrasound examinations in order to overcome the problems described, and in general to avoid interference with an internal organ/tissue not visualized/visualizable with a standard ultrasound scan.

The general object of the present invention is to provide an ultrasound image processing system that overcomes the problems of the prior art.

A specific object of the present invention is to provide an ultrasound image processing system that simplifies the interaction between the medical instrument and ultrasound images.

Another object of the present invention is to provide an ultrasound image processing system that is simple for a medical operator to use.

A further object of the present invention is to provide an ultrasound image processing system that is minimally risky for the patient.

EP 8 530 221 A1, US 2026 / 354152 A1, Y Sato ET AL: "Image guidance of breast cancer surgery using 3-D ultrasound images and augmented reality visualization IEEE Transactions on Medical Imaging, 2 January 1998 (1998-01-02), pages 681-693, XP055468407, UNITED STA TE8 D01: 10.1 109/42. 736019 are related to ultrasound based guidance of a medical instrument.

MARCO SOLBIATI ET AL: "Augmented reality for interventional oncology: proof-of-concept study of a novel high-end guidance system platform", EUROPEAN RADIOLOGY EXPERIMENTAL, vol. 2, no. 1, 31 July 2018 (2018-07-31), XP055769915, D01: 10. 1 186/s41 747-0 1 8-0054-5 is related to the usage of augmented reality during interventional procedures.

### SUMMARY OF THE INVENTION

In a first aspect of the invention, these and other objects are achieved by an ultrasound image processing system according to claim 1.

### SUMMARY OF THE DISCLOSURE

Preferably, said processing unit is further configured to:
- receive, from said camera, said real scene;
- graphically overlay said overall image onto said real scene received, thereby determining an augmented reality overall image;
- send said augmented reality overall image to said image display;
and said image display is adapted to display said augmented reality overall image.

The ultrasound image processing system preferably comprises augmented and virtual reality glasses comprising said camera coupled to said display.

Said first position sensors are preferably provided on said ultrasound instrument. Said ultrasound instrument preferably comprises an ultrasound probe.

The ultrasound image processing system preferably comprises second position sensors provided in fixed positions relative to said ultrasound instrument.

Said third position sensors are preferably provided on said medical instrument. Said medical instrument preferably comprises an interventional needle. Preferably, said virtualized image preferably comprises:
- a first part of said medical instrument that is directly visible and directly acquirable by said camera;
- a second part of the medical instrument that is not directly visible and not directly acquirable by said camera.

A positional reference system is preferably defined by said second position sensors and by respective second position data.

Said georeferenced ultrasound image and said virtualized and georeferenced image of the medical instrument are preferably georeferenced relative to said positional reference system.

In a second aspect of the disclosure, these and other objects are achieved by an ultrasound image processing method comprising the steps of:
providing an ultrasound instrument, in particular adapted to contact a patient's body, coupled to first position sensors;
providing a medical instrument, in particular for insertion into said patient's body, coupled to third position sensors;
providing a camera in data connection with said instruments and adapted to capture a real scene (Img_Real);
providing an image processing device configured to process images originating from said camera and/or from said instruments;
on the part of said an ultrasound instrument, carrying out the steps of:
   - performing an ultrasound scan by emitting an ultrasound beam and determining an ultrasound image, wherein said ultrasound image lies along an ultrasound beam plane;
   - transmitting said ultrasound image to said image processing device;
on the part of said camera carrying out the steps of:
   - picking up first position data of said ultrasound instrument by means of said first position sensors;
   - transmitting said first position data to said image processing device for a corresponding processing;
   - acquiring an image of said medical instrument;
   - picking up third position data of said medical instrument by means of said third position sensors;
   - transmitting said image of said medical instrument and said third position data to said image processing device, for a corresponding processing;
on the part of said image processing device, carrying out the steps of:
   - processing said ultrasound image and said first position data, thereby determining a georeferenced ultrasound image;
   - graphically positioning said georeferenced ultrasound image along said ultrasound projection beam coming out of said ultrasound instrument along said ultrasound beam plane;
   - processing said image of said medical instrument and said third position data, thereby determining a virtualized image of said medical instrument;
   - processing said virtualized image of said medical instrument and said third position data, thereby determining a virtualized and georeferenced image of said medical instrument, wherein said virtualized and georeferenced image lies along a second image plane;
   - overlaying said virtualized and georeferenced image of the medical instrument onto said georeferenced ultrasound image so that said second image plane intersects said ultrasound beam plane;
      the overlay determining an overall image wherein the reciprocal positioning of the entire medical instrument relative to said georeferenced ultrasound image is visible.

Preferably, there are provided the further steps of:
- receiving, from said camera, said real scene;
- graphically overlaying said overall image onto said real scene received, thereby determining an augmented reality overall image;
- sending said augmented reality overall image to said image display;
and, on the part of said image display, carrying out the step of displaying said augmented reality overall image.

Preferably, there is provided a step of providing augmented and virtual reality glasses comprising said camera coupled to said display.

Preferably, there is provided a step of:
- providing said first position sensors on said ultrasound instrument and
- providing said third position sensors on said medical instrument.

The invention as described enables an interaction between a medical instrument and the practice of ultrasound examination such as to overcome the problems illustrated, thus achieving the following technical effects:
- simplification of the interaction between the medical instrument and ultrasound images;
- simplicity of use for a medical operator;
- minimization of risk for the patient.

The stated technical effects/advantages and other technical effects/advantages of the invention will emerge in greater detail from the description that follows, accompanied by an example of an embodiment, given by way of illustration and not limitation with reference to the appended drawings.

### BRIEF DESCRIPTION OF THE DRAWINGS

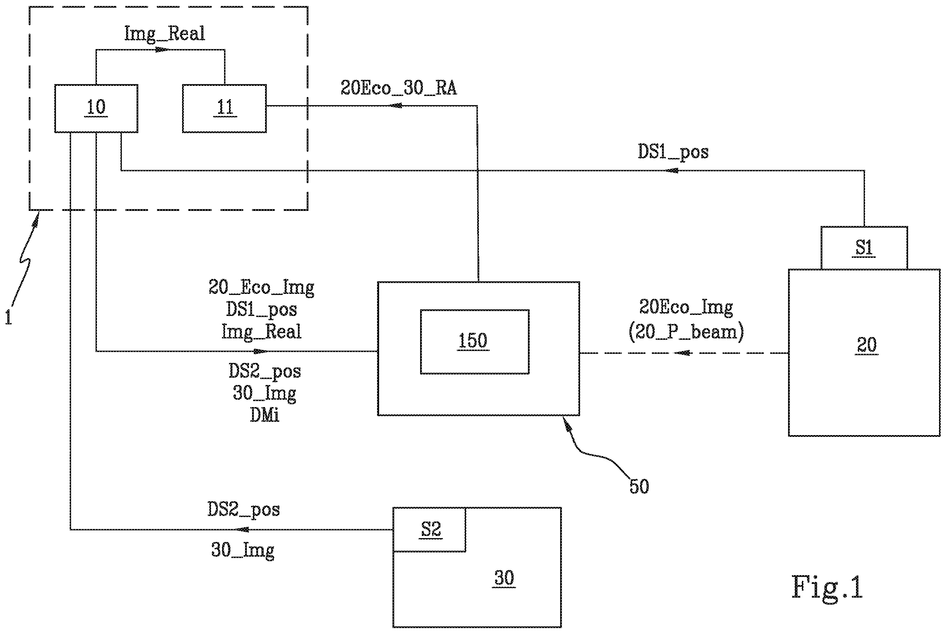

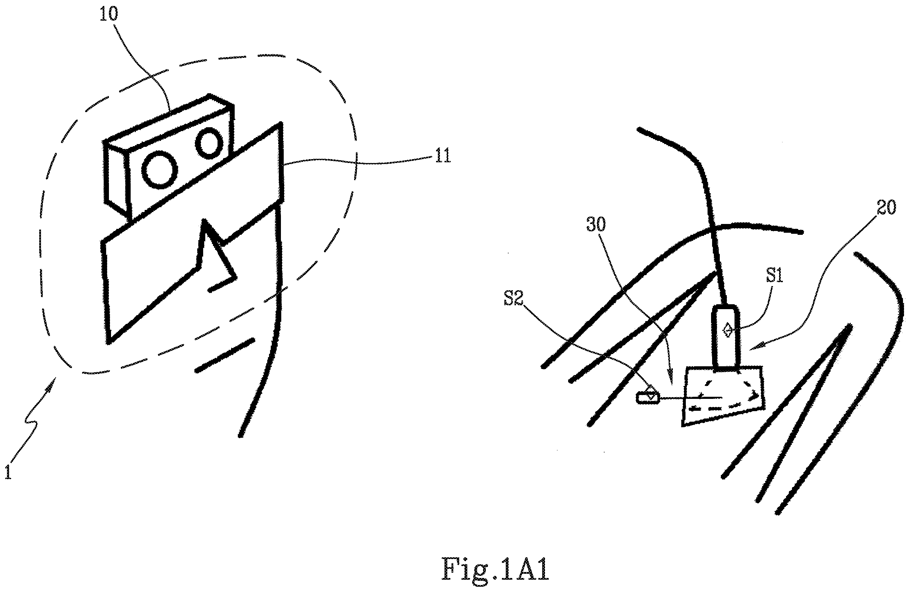

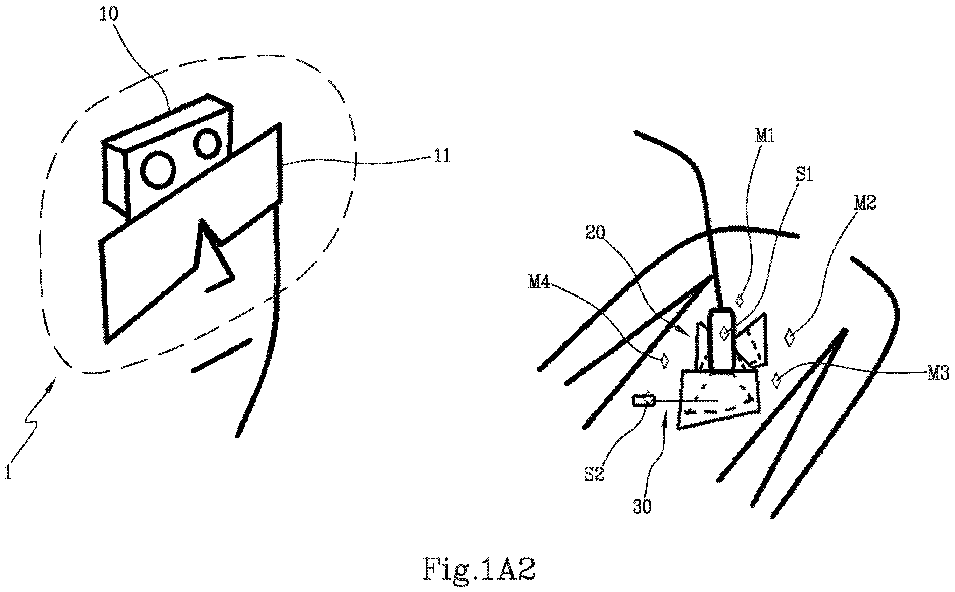

Figure 1 represents in general the ultrasound image processing system according to the invention.
Figure 1A1 is an overall view of one embodiment of the invention with respective sensors integral with an ultrasound instrument and with a medical instrument;
Figure 1A2 is an overall view of a variant of the embodiment of the invention with sensors applied to a patient's body;
Figure 2 shows a detailed view of an image processing device in the system shown in figure 1;
Figure 2A shows several components of the device in figure 2 in a further detail.
Figures 3A and 3B show details of the graphic processing performed by the device in figures 2 and 2A.
Figures 4A and 4B respectively represent a real view (a photo) of one example of the method of the disclosure and a corresponding schematic view.
Figure 5 is a schematic view of the system of the invention.

### DETAILED DESCRIPTION

The invention describes a system for processing ultrasound images and tracing medical instruments.

A first aspect of the invention describes an ultrasound image processing system comprising an ultrasound instrument, a medical instrument, a camera in data connection with said instruments, and an image processing device configured to process images originating from the camera and/or from the instruments in order to define an overall image in which the reciprocal positioning of the entire medical instrument relative to a georeferenced ultrasound image determined by the ultrasound instrument is visible.

With particular reference to figures 1 and 1A, the ultrasound image processing system comprises an ultrasound instrument 20, in particular adapted to contact a patient's body.

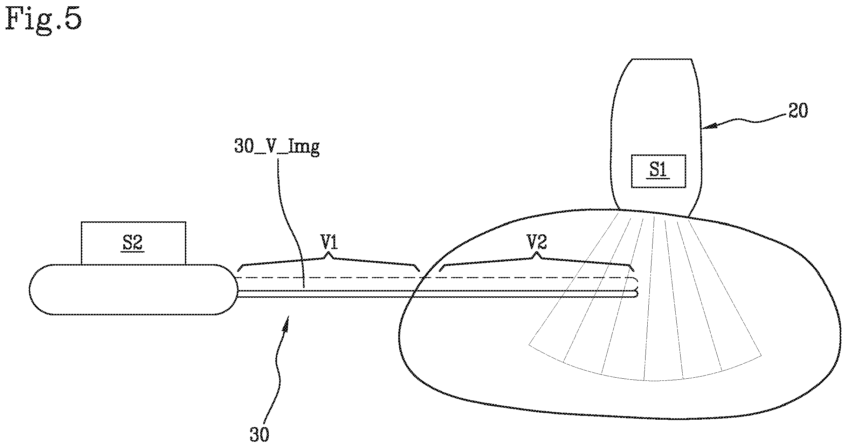

The ultrasound instrument is coupled to first position sensors S1 (Figs. 1 and 5). According to the invention, the first position sensors S1 are provided on the ultrasound instrument 20 and, consequently, are adapted to move integrally with the ultrasound instrument itself (Fig. 1A1).

The technical effect achieved is a real-time detection of the position of the ultrasound instrument and a consequent georeferencing relative to the instrument itself.

In one variant, second position sensors (markers) Mi (i=1..n) are provided, in addition to those coupled to the ultrasound instrument, and also positioned in fixed positions relative to the patient; they are shown as M1, M2, M3 and M4 in figure 1A2.

In other words, the second position sensors Mi are applied to the patient's body. This allows the ultrasound image to be georeferenced relative to the patient's body.

The technical effect achieved is that the ultrasound image picked up is rendered stable relative to the positioning of the second position sensors Mi applied to the patient, also with several repetitions.

Every image acquired by the ultrasound instrument thus becomes referenced and in a stable position relative to the patient's body, also independently of the successive acquisitions by the ultrasound instrument.

The stable referencing operation can be repeated multiple times to render all the stable georeferenced images visible simultaneously.

In a preferred embodiment of the invention, the ultrasound instrument 20 comprises an ultrasound probe.

With particular reference to figure 1, the ultrasound instrument 20 is adapted to perform an ultrasound scan by emitting an ultrasound projection beam and determining an ultrasound image 20_Eco_lmg which lies along an ultrasound beam plane 20_P_beam.

The ultrasound image processing system further comprises a medical instrument 30, in particular adapted to be inserted into the patient's body.

The medical instrument 30 is coupled to third position sensors S2 (Figs. 1 and 5). According to the invention, the third position sensors S2 are provided on said medical instrument 30 and, consequently, are adapted to move integrally with the medical instrument itself.

According to the invention, the third position sensors S2 are positioned on the medical instrument in a position proximal to a handgrip thereof, thus remaining outside the patient's body during a generation of images of interaction between the ultrasound and medical instruments.

In this manner, the third position sensors S2 can be acquired directly by a camera, as described below, as the visual path between the third sensors and the camera itself is in no way obstructed. The technical effect achieved is a real-time detection of the position of the medical instrument.

The tip of the instrument that comes into contact with and penetrates the patient's body is situated in a position distal from the handgrip.

In a preferred embodiment of the invention, the medical instrument 30 comprises an interventional needle.

In particular, the interventional needle is a needle for biopsy/amniocentesis or the like.

With reference to figures 1 and 1A, the ultrasound image processing system of the invention comprises a camera 10 in data connection with the aforesaid medical and ultrasound instruments.

The camera 10, according to the invention, is adapted to capture a real scene (Img_Real) of the ultrasound instrument 20 and of the medical instrument 30 during a reciprocal interaction thereof.

In particular, according to the invention, for the purpose of detecting the reciprocal interaction of said instruments it is not necessary for there to be coplanarity between the reciprocal planes of action.

Associated with the camera 10 there is a display 11 configured to display the images/image stream acquired by the camera.

In one embodiment of the invention, shown in particular in figure 1A, the ultrasound image processing system comprises augmented and virtual reality glasses 1 in turn comprising the camera 10 and the display 11.

The ultrasound image processing system, according to the invention, further comprises an image processing device 50 (Fig.1) configured to process the images Img_Real originating from the camera 10.

The image processing device 50 is further configured to process the images 20_Eco_lmg and 30_Img originating respectively from the ultrasound instrument 20 and from the camera 10.

The image processing device 50 is adapted to receive the ultrasound image 20_Eco_lmg from the ultrasound instrument 20.

In other words, the ultrasound instrument 20 is adapted to transmit the ultrasound image 20_Eco_lmg to the image processing device 50.

According to the invention, the camera 10 is adapted to pick up first position data DS1_pos of the ultrasound instrument 20 by means of the first position sensors S1 and to transmit the first position data DS1_pos to the image processing device 50 for a corresponding processing.

Alternatively or additionally, the camera 10 is adapted to pick up second position data DM1i of the ultrasound instrument 20 by means of the second position sensors Mi and to transmit the second position data DMi to the image processing device 50 for a corresponding processing.

The camera 10 is further adapted to
- acquire an image 30_Img of the medical instrument 30;
- pick up third position data DS2_pos of the medical instrument 30 by means of the third position sensors S2;
- transmit the image 30_Img of the medical instrument 30 and the third position data DS2_pos to the image processing device 50, for a corresponding processing.

According to the invention, the image processing device 50 comprises an electronic processing unit 150 configured to process the images originating from the camera 10 and/or from the instruments 20 and 30.

In the invention presented and in the subsequent claims, the electronic processing unit 150 is presented as divided into distinct functional modules (memory modules or operating modules) for the sole purpose of describing the functions thereof in a clear and complete manner.

The electronic processing unit 150 can consist of a single electronic device, suitably programmed to perform the functions described, and the different modules can correspond to hardware entities and/or routine software forming part of the programmed device.

Alternatively or additionally, said functions can be carried out by a plurality of electronic devices over which the aforesaid functional modules can be distributed. The electronic processing unit 150 can further rely on one or more processors to execute the instructions contained in the memory modules.

The aforesaid functional modules can also be distributed over various local or remote computers based on the architecture of the network they reside in.

The system of the invention enables numerous advantages/technical effects to be obtained for the different individuals involved.

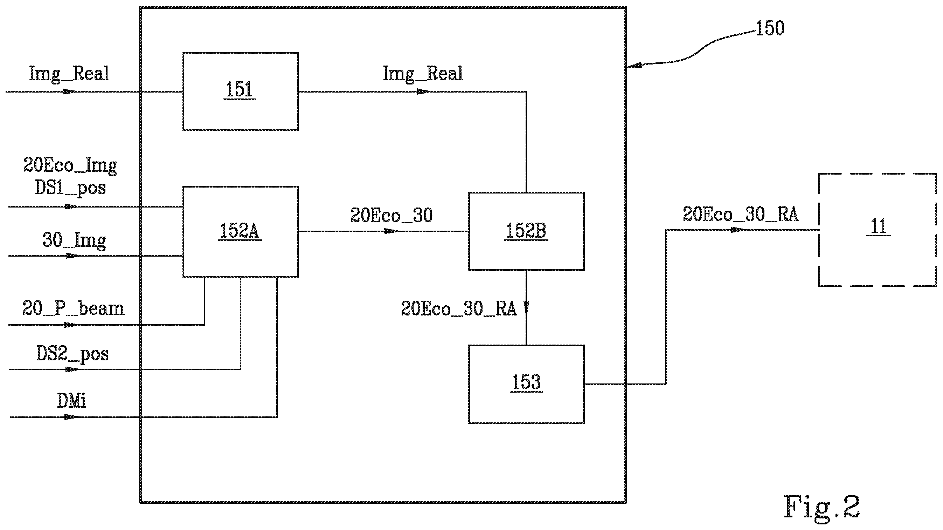

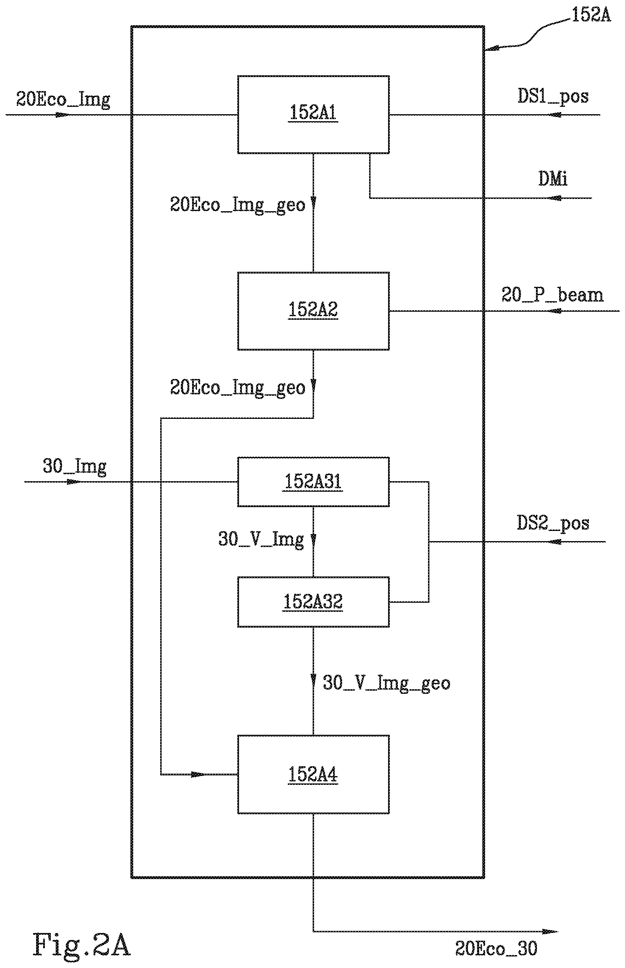

According to the invention, with particular reference to figure 2A, the processing unit 150 is configured to process 152A1 the ultrasound image 20_Eco_lmg and the first position data DS1_pos, thereby determining a georeferenced ultrasound image 20_Eco_lmg_geo.

In other words, the processing unit 150 comprises a first processing module 152A1 configured to process the ultrasound image 20_Eco_lmg and the first position data DS1_pos, thereby determining a georeferenced ultrasound image 20_Eco_lmg_geo.

Preferably, the ultrasound image 20_Eco_lmg_geo is georeferenced relative to a positional reference system Geo_Ref defined by the second position sensors Mi and respective second position data DMi together.

The second position sensors Mi are preferably optical or electromagnetic sensors.

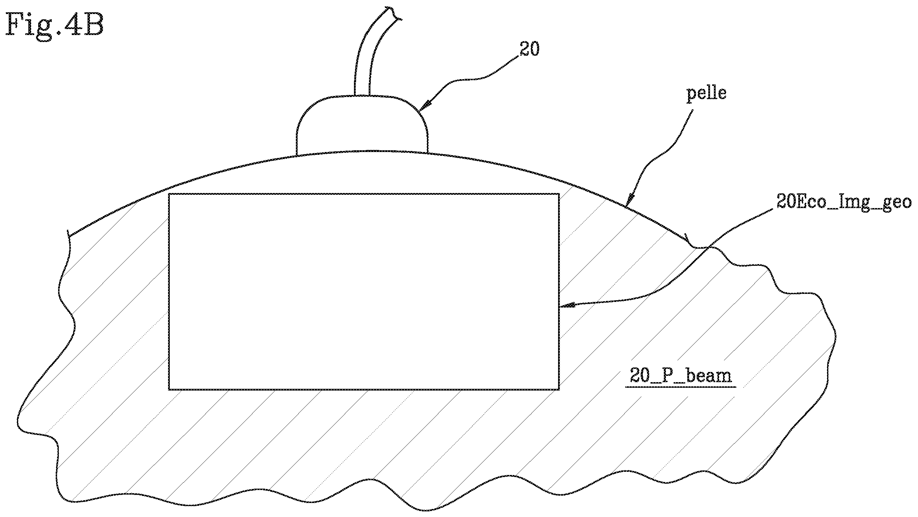

With particular reference to figure 4B, the processing unit 150 is further configured to graphically position 152A2 the georeferenced ultrasound image 20_Eco_lmg_geo along the ultrasound projection beam coming out of the ultrasound instrument 20 along the ultrasound beam plane 20_P_beam, with the optical effect of displaying the georeferenced image 20_Eco_img_geo.

In other words, the processing unit 150 comprises a graphic positioning module 152A2 configured to graphically position the georeferenced ultrasound image 20_Eco_lmg_geo along the ultrasound projection beam coming out of the ultrasound instrument 20 along the ultrasound beam plane 20_P_beam, with the optical effect of displaying the georeferenced image 20_Eco_img_geo.

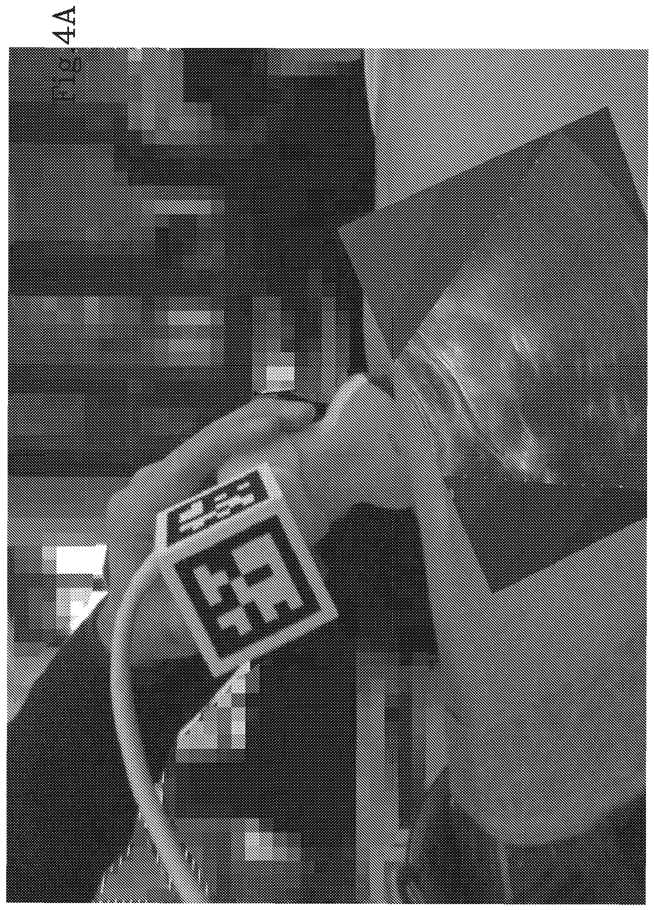

Figure 4a, by contrast, is a photo that provides a real view of the interaction between the ultrasound instrument, the emitted beam and the patient on whom the instrument is used.

Since the first position sensors S1 are positioned so as to remain outside the patient's body, in order to avoid invading the patient's body in any way with said sensors it is necessary to determine a correct theoretical position of the medical instrument inside the body also when it is not visible from the outside.

With particular reference to figure 4B, the processing unit 150 is further configured to process 152A31 the image of the medical instrument 30_Img and the third position data DS2_pos, thereby determining a virtualized image 30_V_Img (Fig. 2A) of the medical instrument 30.

In other words, the processing unit 150 comprises a processing module 152A31 configured to process 152A31 the image of the medical instrument 30_Img and the third position data DS2_pos, thereby determining a virtualized image 30_V_Img of the medical instrument 30.

In other words, with particular reference to figure 5, the virtualized image 30_V_Img comprises:
- a first part V1 of the medical instrument 30 that is directly visible and directly acquirable by the camera 10, i.e. without obstacles; the first part V1 is for example a part of a needle outside the patient's body;
- a second part V2 of the medical instrument 30 that is not directly visible and not directly acquirable by the camera 10, for example, an extension of the needle inside the body, not visible from outside the patient's body.

According to the invention, therefore, the processing module 152A31 is configured to graphically construct the virtualized image 30_V_Img on the basis of the position data picked up by the first position sensors S1 and a predefined conformation conf_30 of the medical instrument 30.

It follows that, in the case of a medical needle provided with first position sensors S1 and having a predefined shape conf_30, a virtualized image 30_V_Img of the needle both inside and outside the body is exactly reconstructed, ensuring that the operator can see the position of the needle in the body and the continuation of the image of the needle outside the body as if the body were open and the needle accessible.

The technical effect achieved is, therefore, that of rendering the position of the needle detectable and evaluable also in areas where this is not physically possible.

The processing unit 150 is further configured to process 152A32 the virtualized image 30_V_Img and the third position data DS2_pos, thereby determining a virtualized and georeferenced image 30_V_Img_geo of the medical instrument 30, wherein the virtualized and georeferenced image 30_V_Img_geo lies along a second image plane P_30_Img.

It may be understood that the second image plane P_30_Img is identified in terms of position in space, as it is the plane in which the virtualized and georeferenced image 30_V_Img_geo lies, in turn identified in terms of position in space by means of the virtualized image 30_V_Img - wherein said virtualized image 30_V_Img is graphically constructed on the basis of the position data picked up by the first position sensors S1 and a predefined conformation conf_30 of the medical instrument 30 - and by means of the third position data DS2_pos (picked up by means of the third position sensors S2).

In particular, the virtualized image 30_V_Img_geo of the medical instrument 30 is georeferenced in reference to the positional reference system Geo_Ref.

In other words, the processing unit 150 comprises a second processing module 152A32 (Fig. 2A) configured to process the virtualized image 30_V_Img and the third position data DS2_pos, thereby determining a virtualized and georeferenced image 30_V_Img_geo of the medical instrument, in particular in reference to the positional reference system Geo_Ref, wherein the virtualized and georeferenced image 30_V_Img_geo lies along the second image plane P_30_lmg.

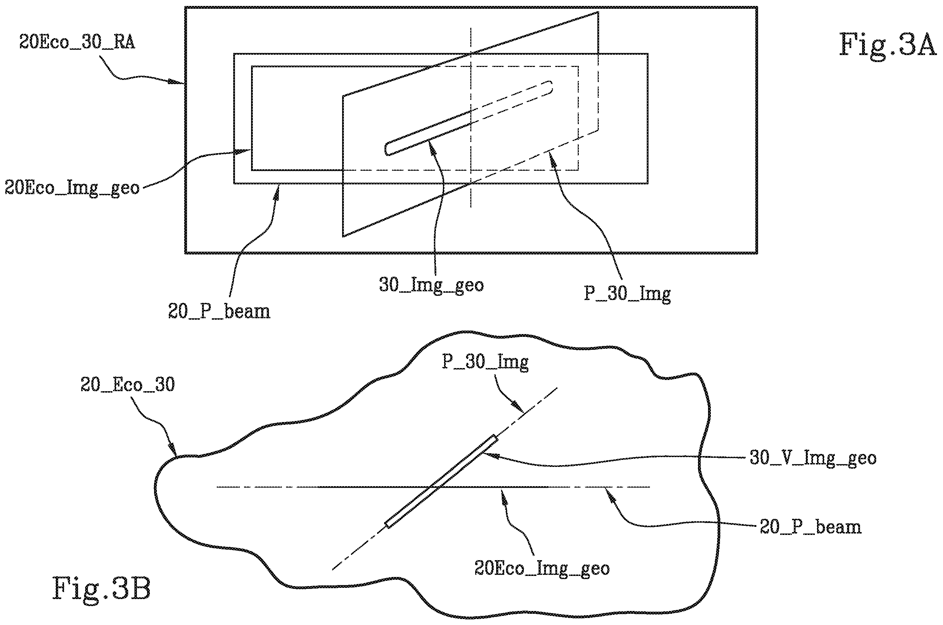

With particular reference to figures 3B and 2A, the processing unit 150 is further configured to overlay 152A4 the virtualized and georeferenced image of the medical instrument 30_V_Img_geo onto the georeferenced ultrasound image 20_Eco_lmg_geo so that the second image plane P_30_lmg intersects the ultrasound beam plane 20_P_beam.

It may be understood that the second image plane P_30_Img intersects the ultrasound beam plane 20_P_beam, since the virtualized and georeferenced image of the medical instrument 30_V_Img_geo is overlaid onto the georeferenced ultrasound image 20_Eco_Img_geo, wherein the virtualized and georeferenced image of the medical instrument 30_V_Img_geo is defined in terms of spatial coordinates by means of the virtualized image 30_V_Img and the third position data DS2_pos, and the georeferenced ultrasound image 20_Eco_lmg_geo is defined in terms of spatial coordinates by means of the ultrasound image 20_Eco_lmg and the first position data DS1_pos; consequently, the intersection between the second image plane P_30_Img and the ultrasound beam plane 20_P_beam is defined in terms of spatial coordinates. In other words, the electronic processing unit 150 comprises an overlay module 152A4 configured to overlay the virtualized and georeferenced image of the medical instrument 30_V_Img_geo onto the georeferenced ultrasound image 20_Eco_lmg_geo so that the second image plane P_30_lmg intersects the ultrasound beam plane 20_P_beam.

The technical effect achieved by said overlay is a determination of an overall image 20Eco_30 (Fig. 3B,2a ) in which the reciprocal positioning of the entire medical instrument 30 relative to the georeferenced ultrasound image 20_Eco_lmg_geo is visible.

The major technical effect of this solution is that the operator always has at his or her disposal the reciprocal positions of the entire medical instrument and of the ultrasound plane and does not need to decide how to move the medical instrument to render it visible inside the body by means of an ultrasound image, as is the case in the prior art, based only on his or her own experience and in the absence of objective references. The operator can thus act upon the medical instrument with a full view of where it is positioned and also in reference to the georeferenced ultrasound image 20_Eco_lmg_geo.

According to the invention, in fact, the needle is entirely visible and virtualized, i.e. both the part thereof outside the patient's body and the one inside the patient's body are visible.

With particular reference to figure 2A, the processing unit 150 is further configured to receive 151, from the camera 10, the real captured scene Img_Real.

In other words, the processing unit 150 comprises a receiving module 151 configured to receive the real captured scene Img_Real from the camera 10.

With particular reference to figure 2, the processing unit 150 is further configured to graphically overlay 152B the overall image 20Eco_30 onto the real scene Img_Real received, thereby determining an augmented reality overall image 20Eco_30_RA (Fig. 3A).

In other words, the processing unit 150 comprises an overlay module 152B configured to graphically overlay the overall image 20Eco_30 onto the real scene Img_Real received, thereby determining the augmented reality overall image 20Eco_30_RA.

Furthermore, with reference to figure 2, the processing unit 150 is further configured to send 153 the augmented reality overall image 20Eco_30_RA (Fig. 3A) to the image display 11.

In other words, the processing unit 150 comprises a sending module 152B configured to send the augmented reality overall image 20Eco_30_RA to the image display 11.

The image display 11 is adapted to display the augmented reality overall image 20Eco_30_RA.

The technical effect achieved is the possibility of seeing, with the glasses 1 or the monitor 10, both the first sensors S1 and the third sensors S2, and of reading the interaction between the ultrasound plane and the medical instrument.

Consequently, when the medical instrument, in particular the needle, disappears into the patient's body, the reciprocal positioning of the needle and of the ultrasound plane is visible all the same.

A second aspect of the disclosure relates to a method for processing ultrasound images, comprising the steps of:
providing an ultrasound instrument 20, in particular adapted to contact a patient's body, coupled to first position sensors S1;
providing a medical instrument 30, in particular for insertion into the patient's body, coupled to third position sensors S2;
providing a camera (10) in data connection with said instruments (20,30) and adapted to capture a real scene Img_Real;
providing an image processing device 50 configured to process images (Img_Real; 20_Eco_lmg; 30_Img) originating from the camera 10 and/or from the instruments 20,30;
on the part of said an ultrasound instrument (20), carrying out the steps of:
   - performing an ultrasound scan by emitting an ultrasound projection beam and determining an ultrasound image 20_Eco_lmg, wherein said ultrasound image lies along an ultrasound beam plane 20_P_beam;
   - transmitting the ultrasound image 20_Eco_lmg to the image processing device 50;
on the part of said camera (10), carrying out the steps of:
   - picking up first position data DS1_pos of the ultrasound instrument 20 by means of the first position sensors S1;
   - transmitting the first position data DS1_pos to the image processing device 50 for a corresponding processing;
   - acquiring an image 30_Img of the medical instrument 30;
   - picking up third position data DS2_pos of the medical instrument 30 by means of the third position sensors S2;
   - transmitting the image 30_Img of the medical instrument and the third position data DS2_pos to the image processing device 50, for a corresponding processing;
on the part of the image processing device 50, carrying out the steps of:
   - processing (152A1) the ultrasound image 20_Eco_lmg and the first position data DS1_pos, thereby determining a georeferenced ultrasound image 20_Eco_lmg_geo, in particular in reference to a positional reference system Geo_Ref;
   - graphically positioning 152A2 the georeferenced ultrasound image 20_Eco_lmg_geo along the ultrasound projection beam coming out of the ultrasound instrument 20 along the ultrasound beam plane 20_P_beam;
   - processing 152A31 the image of the medical instrument 30_Img and the third position data DS2_pos, thereby determining a virtualized image 30_V_Img of the medical instrument;
   - processing (152A32) the image of the medical instrument 30_Img and the third position data DS2_pos thereby determining a virtualized and georeferenced image (30_V_Img_geo) of the medical instrument, in reference to the positional reference system Geo_Ref, wherein the virtualized and georeferenced image 30_V_Img_geo lies along a second image plane (P_30_lmg);
   - overlaying (152A4) the virtualized and georeferenced image of the medical instrument 30_V_Img_geo onto the georeferenced ultrasound image 20_Eco_lmg_geo so that the second image plane P_30_lmg intersects the ultrasound beam plane 20_P_beam;
      the overlay determining an overall image (20Eco_30) in which the reciprocal positioning of the entire medical instrument (30) relative to the georeferenced ultrasound image 20_Eco_lmg_geo is visible.

Preferably, the method comprises the further steps, on the part of the image processing device 50, of:
- receiving 151, from the camera 10, the real scene Img_Real;
- graphically overlaying 152B the overall image 20Eco_30 onto the real scene Img_Real received, thereby determining an augmented reality overall image 20Eco_30_RA;
- sending 153 the augmented reality overall image 20Eco_30_RA to the image display 11;
and, on the part of the image display 11, carrying out the step of displaying the augmented reality overall image 20Eco_30_RA.

The method further comprises the step of providing augmented and virtual reality glasses 1 comprising the camera 10 coupled to the display 11.

Further steps of the method correspond to the previously described functionalities of the ultrasound instrument and/or medical instrument and/or camera and/or image processing device.

It may be understood from the description that, in the system of the invention, the second image plane P_30_Img corresponds to the plane in which the virtualized and georeferenced image 30_V_Img_geo lies, thus being identified in terms of position in space.

It may be understood from the description that, in the system of the invention, the virtualized and georeferenced image 30_V_Img_geo is identified in terms of position in space by means of the virtualized image 30_V_Img and the third position data DS2_pos.

It may be understood from the description that, in the system of the invention, the virtualized image 30_V_Img is graphically constructed on the basis of the position data picked up by the first position sensors S1 and a predefined conformation conf_30 of the medical instrument 30.

It may be understood from the description that, in the system of the invention, the georeferenced ultrasound image 20_Eco_lmg_geo is defined in terms of spatial coordinates by means of the ultrasound image 20_Eco_lmg and the first position data DS1_pos.

It may be understood from the description that, in the system of the invention, the overlay between the virtualized and georeferenced images of the medical instrument 30_V_Img_geo and the georeferenced ultrasound image 20_Eco_lmg_geo determines the intersection between the second image plane P_30_Img and the ultrasound beam plane 20_P_beam, defining the intersection in terms of spatial coordinates.

An inventive system for processing ultrasound images has been described.

The invention as described enables an innovative interaction between a medical instrument and the practice of ultrasound examination, achieving the following technical effects:
- simplification of the interaction between the medical instrument and the ultrasound images produced;
- simplicity of use for a medical operator;
- minimization of risk for the patient.

## Claims

1. An ultrasound image processing system comprising:
an ultrasound instrument (20), in particular adapted to contact a patient's body, coupled to first position sensors (S1);
a medical instrument (30), in particular for insertion into said patient's body, coupled to third position sensors (S2);
a camera (10) in data connection with said instruments (20,30) and adapted to capture a real scene (Img_Real);
an image processing device (50) configured to process images (Img_Real; 20_Eco_lmg; 30_Img) originating from said camera (10) and/or from said instruments (20,30);
wherein:
said ultrasound instrument (20) is adapted to:
- perform an ultrasound scan by emitting an ultrasound beam and determining an ultrasound image (20_Eco_Img), wherein said ultrasound image lies along an ultrasound beam plane (20_P_beam);
- transmit said ultrasound image (20_Eco_lmg) to said image processing device (50);
said camera (10) is adapted to:
- pick up first position data (DS1_pos) of said ultrasound instrument (20) by means of said first position sensors (S1);
- transmit said first position data (DS1_pos) to said image processing device (50) for a corresponding processing;
- acquire an image (30_Img) of said medical instrument (30);
- pick up third position data (DS2_pos) of said medical instrument (30) by means of said third position sensors (S2);
- transmit said image (30_Img) of said medical instrument and said third position data (DS2_pos) to said image processing device (50), for a corresponding processing;
said image processing device (50) comprises a processing unit (150) configured to:
- process (152A1) said ultrasound image (20_Eco_lmg) and said first position data (DS1_pos), thereby determining a georeferenced ultrasound image (20_Eco_lmg_geo);
- graphically position (152A2) said georeferenced ultrasound image (20_Eco_Img_geo) along said ultrasound projection beam coming out of said ultrasound instrument (20) along said ultrasound beam plane (20_P_beam)
wherein said georeferenced ultrasound image (20_Eco_lmg_geo) is defined in terms of spatial coordinates by means of said ultrasound image (20_Eco_Img) and said first position data (DS1 pos);
- process (152A31) said image of said medical instrument (30_Img) and said third position data (DS2_pos), thereby determining a virtualized image (30_V_Img) of said medical instrument;
- process (152A32) said virtualized image (30_V_Img) of said medical instrument (30) and said third position data (DS2_pos), thereby determining a virtualized and georeferenced image (30_V_Img_geo) of said medical instrument, wherein said virtualized and georeferenced image (30_V_Img_geo) lies along a second image plane (P_30_lmg) and is identified in terms of position in space by means of said virtualized image (30_V_Img) and said third position data (DS2 pos),
wherein said second image plane (P 30 Img) corresponds to the plane in which said virtualized and georeferenced image (30_V_Img_geo) lies, thus being identified in terms of position in space;
- overlay (152A4) said virtualized and georeferenced image of the medical instrument (30_V_Img_geo) onto said georeferenced ultrasound image (20_Eco_lmg_geo) so that said second image plane (P_30_Img) intersects said ultrasound beam plane (20_P_beam),
wherein said overlay between said virtualized and georeferenced image (30_V_Img_geo) of the medical instrument and said georeferenced ultrasound image (20_Eco_lmg_geo) determines said intersection between said second image plane (P 30 Img) and said ultrasound beam plane (20 P beam), thus defining said intersection in terms of spatial coordinates;
the overlay determining an overall image (20Eco_30) in which the reciprocal positioning of the entire medical instrument (30) relative to said georeferenced ultrasound image (20_Eco_Img_geo) is visible.

2. The image processing system according to claim 1 wherein said processing unit (150) is further configured to:
- receive (151), from said camera (10), said real scene (Img_Real);
- graphically overlay (152B) said overall image (20Eco_30) onto said real scene (Img_Real) received, thereby determining an augmented reality overall image (20Eco_30_RA);
- send (153) said augmented reality overall image (20Eco_30_RA) to said image display (11);
and said image display (11) is adapted to display said augmented reality overall image (20Eco_30_RA).

3. The system according to claim 1 or 2, comprising augmented and virtual reality glasses (1) comprising said camera (10) coupled to said display (11).

4. The system according to any one of the preceding claims, wherein said first position sensors (S1) are provided on said ultrasound instrument (20).

5. The system according to any one of the preceding claims, wherein said ultrasound instrument (20) comprises an ultrasound probe.

6. The system according to any one of the preceding claims comprising second position sensors (Mi) provided in fixed positions relative to said ultrasound instrument (20) and said camera (10) is adapted to:
- pick up second position data (DMi) of said ultrasound instrument (20) by means of said second position sensors (Mi).

7. The system according to any one of the preceding claims, wherein said third position sensors (S2) are provided on said medical instrument (30).

8. The system according to any one of the preceding claims, wherein said medical instrument (30) comprises an interventional needle.

9. The system according to any one of the preceding claims, wherein said virtualized image (30_V_Img) comprises:
- a first part (V1) of said medical instrument (30) that is directly visible and directly acquirable by said camera (10);
- a second part (V2) of the medical instrument (30) that is not directly visible and not directly acquirable by said camera (10).

10. The system according to any one of claims 6 to 9, wherein a positional reference system (Geo_Ref) is defined by said second position sensors (Mi) and by respective second position data (DMi).

11. The system according to claim 10, wherein said georeferenced ultrasound image (20_Eco_lmg_geo) and said virtualized and georeferenced image of the medical instrument (30_V_Img_geo) are georeferenced relative to said positional reference system (Geo_Ref).

12. The system according to claim 11, wherein said virtualized image (30_V_Img) is graphically constructed on the basis of the position data detected by the first position sensors (S1) and a predefined conformation (conf 30) of the medical instrument (30).

## Patentansprüche

1. Ultraschallbildverarbeitungssystem, umfassend:
ein Ultraschallinstrument (20), insbesondere dazu geeignet, mit dem Körper eines Patienten in Kontakt zu treten, gekoppelt mit ersten Positionssensoren (S1);
ein medizinisches Instrument (30), insbesondere zum Einführen in den Körper des Patienten, gekoppelt mit dritten Positionssensoren (S2);
eine Kamera (10), die in Datenverbindung mit den Instrumenten (20, 30) steht und dazu eingerichtet ist, eine reale Szene (Img_Real) zu erfassen;
eine Bildverarbeitungsvorrichtung (50), die dazu konfiguriert ist, Bilder (Img_Real; 20_Eco_Img; 30_Img) zu verarbeiten, die von der Kamera (10) und/oder von den Instrumenten (20, 30) stammen;
wobei:
das Ultraschallinstrument (20) dazu eingerichtet ist:
- eine Ultraschallabtastung durch Aussenden eines Ultraschallstrahls durchzuführen und ein Ultraschallbild (20_Eco_lmg) zu bestimmen, wobei das Ultraschallbild entlang einer Ultraschallstrahlebene (20_P_beam) liegt;
- das Ultraschallbild (20_Eco_lmg) an die Bildverarbeitungsvorrichtung (50) zu übertragen;
die Kamera (10) dazu eingerichtet ist:
- erste Positionsdaten (DS1_pos) des Ultraschallinstruments (20) mittels der ersten Positionssensoren (S1) aufzunehmen;
- die ersten Positionsdaten (DS1_pos) an die Bildverarbeitungsvorrichtung (50) zur entsprechenden Verarbeitung zu übertragen;
- ein Bild (30_Img) des medizinischen Instruments (30) zu erfassen;
- dritte Positionsdaten (DS2_pos) des medizinischen Instruments (30) mittels der dritten Positionssensoren (S2) aufzunehmen;
- das Bild (30_Img) des medizinischen Instruments (30) und die dritten Positionsdaten (DS2_pos) an die Bildverarbeitungsvorrichtung (50) zur entsprechenden Verarbeitung zu übertragen;
die Bildverarbeitungsvorrichtung (50) eine Verarbeitungseinheit (150) umfasst, die konfiguriert ist zum:
- Verarbeiten (152A1) des Ultraschallbildes (20_Eco_Img) und der ersten Positionsdaten (DS1_pos), wodurch ein georeferenziertes Ultraschallbild (20_Eco_Img_geo) bestimmt wird;
- graphischen Positionieren (152A2) des georeferenzierten Ultraschallbildes (20_Eco_lmg_geo) entlang des Ultraschallprojektionsstrahls, der aus dem Ultraschallinstrument (20) entlang der Ultraschallstrahlebene (20_P_beam) austritt,
wobei das georeferenzierte Ultraschallbild (20_Eco_Img_geo) in Form räumlicher Koordinaten mittels des Ultraschallbildes (20_Eco_lmg) und der ersten Positionsdaten (DS1_pos) definiert ist;
- Verarbeiten (152A31) des Bildes des medizinischen Instruments (30_Img) und der dritten Positionsdaten (DS2_pos), wodurch ein virtualisiertes Bild (30_V_Img) des medizinischen Instruments bestimmt wird;
- Verarbeiten (152A32) des virtualisierten Bildes (30_V_Img) des medizinischen Instruments (30) und der dritten Positionsdaten (DS2_pos), wodurch ein virtualisiertes und georeferenziertes Bild (30_V_Img_geo) des medizinischen Instruments bestimmt wird, wobei das virtualisierte und georeferenzierte Bild (30_V_Img_geo) entlang einer zweiten Bildebene (P_30_Img) liegt und hinsichtlich seiner Position im Raum mittels des virtualisierten Bildes (30_V_Img) und der dritten Positionsdaten (DS2_pos) identifiziert wird,
wobei die zweite Bildebene (P_30_Img) der Ebene entspricht, in der das virtualisierte und georeferenzierte Bild (30_V_Img_geo) liegt, und somit hinsichtlich der Position im Raum identifiziert wird;
- Überlagern (152A4) des virtualisierten und georeferenzierten Bildes des medizinischen Instruments (30_V_Img_geo) mit dem georeferenzierten Ultraschallbild (20_Eco_Img_geo), so dass die zweite Bildebene (P_30_Img) die Ultraschallstrahlebene (20_P_beam) schneidet,
wobei die Überlagerung zwischen dem virtualisierten und georeferenzierten Bild (30_V_Img_geo) des medizinischen Instruments und dem georeferenzierten Ultraschallbild (20_Eco_Img_geo) den Schnitt zwischen der zweiten Bildebene (P_30_Img) und der Ultraschallstrahlebene (20_P_beam) bestimmt und dadurch diesen Schnitt in Form räumlicher Koordinaten definiert;
die Überlagerung ein Gesamtbild (20Eco_30) bestimmt, in dem die gegenseitige Positionierung des gesamten medizinischen Instruments (30) relativ zu dem georeferenzierten Ultraschallbild (20_Eco_Img_geo) sichtbar ist.

2. Bildverarbeitungssystem nach Anspruch 1, wobei die Verarbeitungseinheit (150) ferner konfiguriert ist zum:
- Empfangen (151), von der Kamera (10), der realen Szene (Img_Real);
- graphischen Überlagern (152B) des Gesamtbildes (20Eco_30) mit der empfangenen realen Szene (Img_Real), wodurch ein Augmented-Reality-Gesamtbild (20Eco_30_RA) bestimmt wird;
- Senden (153) des Augmented-Reality-Gesamtbildes (20Eco_30_RA) an die Bildanzeige (11);
und wobei die Bildanzeige (11) dazu eingerichtet ist, das Augmented-Reality-Gesamtbild (20Eco_30_RA) anzuzeigen.

3. System nach Anspruch 1 oder 2, umfassend Augmented- und Virtual-Reality-Brillen (1), die die Kamera (10) umfassen, welche mit der Anzeige (11) gekoppelt ist.

4. System nach einem der vorhergehenden Ansprüche, wobei die ersten Positionssensoren (S1) an dem Ultraschallinstrument (20) vorgesehen sind.

5. System nach einem der vorhergehenden Ansprüche, wobei das Ultraschallinstrument (20) eine Ultraschallsonde umfasst.

6. System nach einem der vorhergehenden Ansprüche, umfassend zweite Positionssensoren (Mi), die in festen Positionen relativ zu dem Ultraschallinstrument (20) vorgesehen sind, und wobei die Kamera (10) dazu eingerichtet ist:
- zweite Positionsdaten (DMi) des Ultraschallinstruments (20) mittels der zweiten Positionssensoren (Mi) aufzunehmen.

7. System nach einem der vorhergehenden Ansprüche, wobei die dritten Positionssensoren (S2) an dem medizinischen Instrument (30) vorgesehen sind.

8. System nach einem der vorhergehenden Ansprüche, wobei das medizinische Instrument (30) eine interventionelle Nadel umfasst.

9. System nach einem der vorhergehenden Ansprüche, wobei das virtualisierte Bild (30_V_Img) umfasst:
- einen ersten Teil (V1) des medizinischen Instruments (30), der direkt sichtbar und direkt durch die Kamera (10) erfassbar ist;
- einen zweiten Teil (V2) des medizinischen Instruments (30), der nicht direkt sichtbar und nicht direkt durch die Kamera (10) erfassbar ist.

10. System nach einem der Ansprüche 6 bis 9, wobei ein Positionsreferenzsystem (Geo_Ref) durch die zweiten Positionssensoren (Mi) und durch jeweilige zweite Positionsdaten (DMi) definiert ist.

11. System nach Anspruch 10, wobei das georeferenzierte Ultraschallbild (20_Eco_Img_geo) und das virtualisierte und georeferenzierte Bild des medizinischen Instruments (30_V_Img_geo) relativ zu dem Positionsreferenzsystem (Geo_Ref) georeferenziert sind.

12. System nach Anspruch 11, wobei das virtualisierte Bild (30_V_Img) auf Grundlage der von den ersten Positionssensoren (S1) erfassten Positionsdaten und einer vordefinierten Ausgestaltung (conf 30) des medizinischen Instruments (30) graphisch konstruiert wird.

## Revendications

1. Système de traitement d'images ultrasonores comprenant :
un instrument ultrasonore (20), en particulier adapté pour entrer en contact avec le corps d'un patient, couplé à des premiers capteurs de position (S1) ;
un instrument médical (30), en particulier destiné à être inséré dans ledit corps du patient, couplé à des troisièmes capteurs de position (S2) ;
une caméra (10) en liaison de données avec lesdits instruments (20, 30) et adaptée pour capturer une scène réelle (Img_Real) ;
un dispositif de traitement d'images (50) configuré pour traiter des images (Img_Real ; 20_Eco_lmg ; 30_Img) provenant de ladite caméra (10) et/ou desdits instruments (20, 30) ;
dans lequel :
ledit instrument ultrasonore (20) est adapté pour :
- effectuer un balayage ultrasonore en émettant un faisceau ultrasonore et déterminer une image ultrasonore (20_Eco_Img), ladite image ultrasonore se trouvant le long d'un plan de faisceau ultrasonore (20_P_beam) ;
- transmettre ladite image ultrasonore (20_Eco_Img) audit dispositif de traitement d'images (50) ;
ladite caméra (10) est adaptée pour :
- relever des premières données de position (DS1_pos) dudit instrument ultrasonore (20) au moyen desdits premiers capteurs de position (S1) ;
- transmettre lesdites premières données de position (DS1_pos) audit dispositif de traitement d'images (50), pour un traitement correspondant ;
- acquérir une image (30_Img) dudit instrument médical (30) ;
- relever des troisièmes données de position (DS2_pos) dudit instrument médical (30) au moyen desdits troisièmes capteurs de position (S2) ;
- transmettre ladite image (30_Img) dudit instrument médical (30) et lesdites troisièmes données de position (DS2_pos) audit dispositif de traitement d'images (50), pour un traitement correspondant ;
ledit dispositif de traitement d'images (50) comprend une unité de traitement (150) configurée pour :
- traiter (152A1) ladite image ultrasonore (20_Eco_lmg) et lesdites premières données de position (DS1_pos), déterminant ainsi une image ultrasonore géoréférencée (20_Eco_lmg_geo) ;
- positionner graphiquement (152A2) ladite image ultrasonore géoréférencée (20_Eco_Img_geo) le long dudit faisceau de projection ultrasonore sortant dudit instrument ultrasonore (20) le long dudit plan de faisceau ultrasonore (20_P_beam),
dans lequel ladite image ultrasonore géoréférencée (20_Eco_Img_geo) est définie en termes de coordonnées spatiales au moyen de ladite image ultrasonore (20_Eco_Img) et desdites premières données de position (DS1_pos) ;
- traiter (152A31) ladite image dudit instrument médical (30_Img) et lesdites troisièmes données de position (DS2_pos), déterminant ainsi une image virtualisée (30_V_Img) dudit instrument médical;
- traiter (152A32) ladite image virtualisée (30_V_Img) dudit instrument médical (30) et lesdites troisièmes données de position (DS2_pos), déterminant ainsi une image virtualisée et géoréférencée (30_V_Img_geo) dudit instrument médical, dans lequel ladite image virtualisée et géoréférencée (30_V_Img_geo) se trouve le long d'un second plan d'image (P_30_Img) et est identifiée en termes de position dans l'espace au moyen de ladite image virtualisée (30_V_Img) et desdites troisièmes données de position (DS2_pos),
dans lequel ledit second plan d'image (P_30_Img) correspond au plan dans lequel se trouve ladite image virtualisée et géoréférencée (30_V_Img_geo), étant ainsi identifié en termes de position dans l'espace ;
- superposer (152A4) ladite image virtualisée et géoréférencée de l'instrument médical (30_V_Img_geo) sur ladite image ultrasonore géoréférencée (20_Eco_Img_geo) de sorte que ledit second plan d'image (P_30_Img) coupe ledit plan de faisceau ultrasonore (20_P_beam),
dans lequel ladite superposition entre ladite image virtualisée et géoréférencée (30_V_Img_geo) de l'instrument médical et ladite image ultrasonore géoréférencée (20_Eco_Img_geo) détermine ladite intersection entre ledit second plan d'image (P_30_Img) et ledit plan de faisceau ultrasonore (20_P_beam), définissant ainsi ladite intersection en termes de coordonnées spatiales ;
la superposition déterminant une image globale (20Eco_30) dans laquelle le positionnement réciproque de l'ensemble de l'instrument médical (30) par rapport à ladite image ultrasonore géoréférencée (20_Eco_Img_geo) est visible.

2. Système de traitement d'images selon la revendication 1, dans lequel ladite unité de traitement (150) est en outre configurée pour :
- recevoir (151), de ladite caméra (10), ladite scène réelle (Img_Real) ;
- superposer graphiquement (152B) ladite image globale (20Eco_30) sur ladite scène réelle (Img_Real) reçue, déterminant ainsi une image globale en réalité augmentée (20Eco_30_RA) ;
- envoyer (153) ladite image globale en réalité augmentée (20Eco_30_RA) audit afficheur d'images (11) ;
et ledit afficheur d'images (11) est adapté pour afficher ladite image globale en réalité augmentée (20Eco_30_RA).

3. Système selon la revendication 1 ou 2, comprenant des lunettes de réalité augmentée et virtuelle (1) comprenant ladite caméra (10) couplée audit afficheur (11).

4. Système selon l'une quelconque des revendications précédentes, dans lequel lesdits premiers capteurs de position (S1) sont prévus sur ledit instrument ultrasonore (20).

5. Système selon l'une quelconque des revendications précédentes, dans lequel ledit instrument ultrasonore (20) comprend une sonde ultrasonore.

6. Système selon l'une quelconque des revendications précédentes comprenant des seconds capteurs de position (Mi) disposés dans des positions fixes par rapport audit instrument ultrasonore (20), et ladite caméra (10) est adaptée pour :
- relever des secondes données de position (DMi) dudit instrument ultrasonore (20) au moyen desdits seconds capteurs de position (Mi).

7. Système selon l'une quelconque des revendications précédentes, dans lequel lesdits troisièmes capteurs de position (S2) sont prévus sur ledit instrument médical (30).

8. Système selon l'une quelconque des revendications précédentes, dans lequel ledit instrument médical (30) comprend une aiguille interventionnelle.

9. Système selon l'une quelconque des revendications précédentes, dans lequel ladite image virtualisée (30_V_Img) comprend :
- une première partie (V1) dudit instrument médical (30) qui est directement visible et directement acquérable par ladite caméra (10) ;
- une seconde partie (V2) de l'instrument médical (30) qui n'est pas directement visible et n'est pas directement acquérable par ladite caméra (10).

10. Système selon l'une quelconque des revendications 6 à 9, dans lequel un système de référence positionnel (Geo_Ref) est défini par lesdits seconds capteurs de position (Mi) et par des secondes données de position respectives (DMi).

11. Système selon la revendication 10, dans lequel ladite image ultrasonore géoréférencée (20_Eco_Img_geo) et ladite image virtualisée et géoréférencée de l'instrument médical (30_V_Img_geo) sont géoréférencées par rapport audit système de référence positionnel (Geo_Ref).

12. Système selon la revendication 11, dans lequel ladite image virtualisée (30_V_Img) est construite graphiquement sur la base des données de position détectées par les premiers capteurs de position (S1) et d'une conformation prédéfinie (conf 30) de l'instrument médical (30).
